# EUROPEAN PATENT APPLICATION

(11) **EP 1 876 163 A1**
(43) Date of publication of application: **09.01.2008**
(21) Application number: 06745694.7
(22) Date of filing: 26.04.2006
(51) Int. Cl.: C07C 41/28, C07C 43/15

(54) **METHOD FOR PRODUCING UNSATURATED ETHER COMPOUND**

(30) Priority: 26.04.2005 JP 2005127352
(71) Applicant: Kyowa Hakko Chemical Co., Ltd., Tokyo 103-0022 (JP)
(72) Inventor: ITO, Katsuhiro, 3, Daikyo-cho 2-chome, Yokkaichi-shi, Mie 510-8502 (JP); NUMAZAKI, Ryo, 3, Daikyo-cho 2-chome, Yokkaichi-shi, Mie 510-8502 (JP); HOTTA, Iwao, 3, Daikyo-cho 2-chome, Yokkaichi-shi, Mie 510-8502 (JP); MATSUOKA, Hiroshi, Nihonbashi Muromachi 3-chome,Chuo-ku,Tokyo 1030022 (JP)
(74) Representative: Dossmann, Gérard
(86) International application number: PCT/JP2006/308716
(87) International publication number: WO 2006/115265

(57) **Abstract**

The present invention provides a method for producing an unsaturated ether compound represented by general formula (2) below, the method comprising reacting an acetal represented by general formula (1) below in the presence of an aliphatic or aromatic sulfonic acid and an organic base having a total carbon number of 3 to 7. (In the formulae, R¹, R², and R³ may be the same or different and each represent substituted or unsubstituted alkyl, substituted or unsubstituted aryl, or substituted or unsubstituted aralkyl, or R¹ and R² form cycloalkyl together with the adjacent carbon atom.)

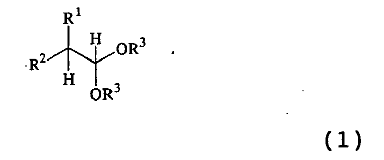

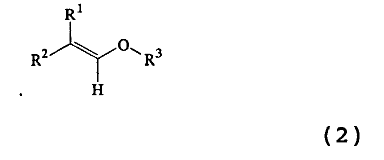

## Description

### Technical Field

The present invention relates to a method for producing an unsaturated ether compound.

### Background Art

There has been known a method for producing an unsaturated ether compound by reacting an acetal in the presence of a catalyst composed of p-toluenesulfonic acid and quinoline. However, the attained yield is about 40% to 70% depending on the unsaturated ether compound to be produced (refer to, for example, Non-Patent Document 1).
In order to resolve the problem, there is disclosed a method for producing an unsaturated ether compound characterized by reacting a corresponding acetal in the presence of a catalyst composed of an acid and an amine. However, in this method, usable amines are limited to high-boiling-point amines in order to avoid distillation of amines, and the reaction should be performed at a very high temperature. Therefore, the method cannot be applied to low-boiling-point acetals (refer to, for example, Patent Document 1).

There is also disclosed a method for producing an allyl vinyl ether from acetaldehyde diallyl acetal characterized by a reactive distillation process in which triethyl amine is allowed to coexist in the presence of a phosphoric acid catalyst in a distillation column (refer to, for example, Patent Document 2).
Patent Document 1: Specification of Patent No. 2505670
Patent Document 2: Specification of Patent No. 2651621
Non-patent Document 1: Chem. Abstr., 1960, Vol. 54, 24452g

### Disclosure of Invention

### Problem to be Solved by the Invention

An object of the present invention is to provide a method for producing an unsaturated ether compound which is capable of by suppressing the formation of by-products and producing the unsaturated ether compound in a high yield. Means for Solving the Problem

The present invention provides the following [1] and [2]:
[1] A method for producing an unsaturated ether compound represented by general formula (2):

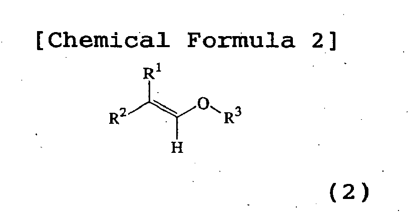

(wherein R¹, R², and R³ may be the same or different and each represent substituted or unsubstituted alkyl, substituted or unsubstituted aryl, or substituted or unsubstituted aralkyl, or R¹ and R² form cycloalkyl together with the adjacent carbon atom), which comprises the reacting acetal represented by general formula (1):

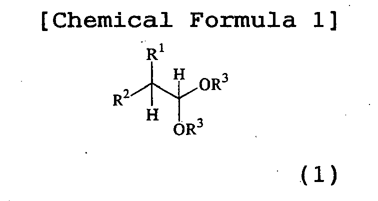

(wherein R¹, R², and R³ are each defined as the same as the above) in the presence of an aliphatic or aromatic sulfonic acid and an organic base having total carbon number 3 to 7.
[2] The method according to [1], wherein the organic base is a tertiary amine.
   Hereinafter, an acetal represented by general formula (1) may be referred to as an "acetal", and an unsaturated ether compound represented by general formula (2) may be referred to as an "unsaturated ether compound".

### Effect of the Invention

The present invention provides a method for producing an unsaturated ether compound which is capable of suppressing the production of by-products and producing the unsaturated ether compound in a high yield.

### Best Mode for Carrying Out the Invention

In the definition of each group in the general formulae, the alkyl means linear or branched alkyl having 1 to 18 carbon atoms, and specific example thereof include methyl, ethyl, propyl, isopropyl, butyl, isobutyl, sec-butyl, tert-butyl, pentyl, hexyl, heptyl, octyl, nonyl, decyl, dodecyl, and octadecyl. In particular, alkyl having 1 to 6 carbon atoms is preferred, and alkyl having 1 to 3 carbon atoms is more preferred.

The cycloalkyl formed by R¹ and R² together with the adjacent carbon atom means cycloalkyl having 3 to 8 carbon atoms, and specific examples thereof include cyclopropyl, cyclobutyl, cyclopentyl, cyclohexyl, cycloheptyl, and cyclooctyl.
The aryl means aryl having 6 to 14 carbon atoms, specific examples thereof include phenyl and naphthyl.

The aralkyl means aralkyl having 7 to 15 carbon atoms, and specific examples thereof include benzyl, phenethyl, naphthylmethyl, and naphthylethyl.
Examples of the substituent of alkyl include alkoxy, alkanoyl, cyano, nitro, halogen atoms, and alkoxycarbonyl.
Examples of alkyl moieties in alkoxy and alkoxycarbonyl include the same as described above for the alkyl.

The alkanoyl means linear or branched alkanoyl having 2 to 7 carbon atoms, specific examples thereof acetyl, and propionyl, butyryl, isobutyryl, valeryl, isovaleryl, pivaloyl, hexanoyl, and heptanoyl.
The halogen atoms means fluorine atom, chlorine atom, bromine atom, and iodine atom.
Examples of substituents of aryl and aralkyl include alkyl, alkoxy, alkanoyl, cyano, nitro, halogen atoms, and alkoxycarbonyl. Examples of the alkyl, alkoxy, alkanoyl, halogen atoms, and alkoxycarbonyl include the same as described above.

Preferred examples of aliphatic or aromatic sulfonic acid include methanesulfonic acid, trifluoromethanesulfonic acid, benzenesulfonic acid, toluenesulfonic acid, and p-toluenesulfonic acid.
As the organic base having a total of 3 to 7 carbon atoms, primary to tertiary amines (linear or branched aliphatic amines and alicyclic amines) and heterocyclic compounds are preferred, and, among these amines, tertiary amines, particularly triethylamine, are particularly preferred.

Examples of a primary amine include n-propylamine, isopropylamine, n-butylamine, isobutylamine, sec-butylamine, tert-butylamine, n-pentylamine, isopentylamine, 1-methylbutylamine, 2-methylbutylamine, 2,3-dimethylpropylamine, tert-pentylamine, cyclopentylamine, n-hexylamine, isohexylamine, 1-methylpentylamine, 2-methylpentylamine, 3-methylpentylamine, 1,2-dimethylbutylamine, 1,3-dimethylbutylamine, 2,3-dimethylbutylamine, 1-ethylbutylamine, 2-ethylbutylamine, 3-ethylbutylamine, 1-methylcyclopentylamine, 2-methylcyclopentylamine, 3-methylcyclopentylamine, cyclohexylamine, n-heptylamine, 1-methylhexylamine, 2-methylhexylamine, 3-methylhexylamine, 4-methylhexylamine, 5-methylhexylamine, 1-ethylpentylamine, 2-ethylpentylamine, 3-ethylpentylamine, 4-ethylpentylamine, 1-propylbutylamine, 1,2-dimethylpentylamine, 1,3-dimethylpentylamine, 1,4-dimethylpentylamine, 2,3-dimethylpentylamine, 2,4-dimethylpentylamine, 1-ethyl-2-methylbutylamine, 1-ethyl-3-methylbutylamine, 2-ethyl-1-methylbutylamine, 1-(1-methylethyl)butylamine, 1,2-dimethylcyclopentylamine, 1,3-dimethylcyclopentylamine, 2,3-dimethylcyclopentylamine, 2,4-dimethylcyclopentylamine, 2,5-dimethylcyclopentylamine, 3,4-dimethylcyclopentylamine, 1-ethylcyclopentylamine, 2-ethylcyclopentylamine, 3-ethylcyclopentylamine, 1-methylcyclohexylamine, 2-methylcyclohexylamine, 3-methylcyclohexylamine, and 4-methylcyclohexylamine. Examples of a secondary amine include dimethylamine, N-methylethylamine, N-methyl-n-propylamine, N-methylisopropylamine, N-methyl-n-butylamine, N-methyl-sec-butylamine, N-methyl-n-pentylamine, N-methylisopentylamine, N-methyl-(1-methylbutyl)amine, N-methyl-(2-methylbutyl)amine, N-methyl-(2,3-dimethylpropyl)amine, N-methyl-(tert-pentyl)amine, N-methyl-cyclopentylamine, N-methyl-n-hexylamine, N-methylisohexylamine, N-methyl-(1-methylpentyl)amine, N-methyl-(2-methylpentyl)amine, N-methyl-(3-methylpentyl)amine, N-methyl-(-1,2-dimethylbutyl)amine, N-methyl-(1,3-dimethylbutyl)amine, N-methyl-(2,3-dimethylbutyl)amine, N-methyl-(1-ethylbutyl)amine, N-methyl-(2-ethylbutyl)amine, N-methyl-(3-ethylbutyl)amine, N-methyl-(1-methylcyclopentyl)amine, N-methyl-(2-methylcyclopentyl)amine, N-methyl-(3-methylcyclopentyl)amine, N-methylcyclohexylamine, diethylamine, N-ethylisopropylamine, N-ethyl-n-butylamine, N-ethyl-sec-butylamine, N-ethyl-tert-butylamine, N-ethyl-n-pentylamine, N-ethylisopentylamine, N-ethyl-(1-methylbutyl)amine, N-ethyl-(2,-methylbutyl)amine, N-ethyl-(2,3-dimethylpropyl)amine, N-ethyl-(tert-pentyl)amine, N-ethylcyclopentylamine, di-n-propylamine, N-n-propylisopropylamine, N-n-propyl-n-butylamine, N-n-propyl-sec-butylamine, N-n-propyl-tert-butylamine, diisopropylamine, N-isopropyl-n-butylamine, N-isopropyl-sec-butylamine, and N-isopropyl-tert-butylamine. Examples of a tertiary amine include trimethylamine, dimethylethylamine, dimethyl-n-propylamine, dimethylisopropylamine, dimethyl-n-butylamine, dimethylisobutylamine, dimethyl-sec-butylamine, dimethyl-tert-butylamine, dimethyl-n-pentylamine, dimethylisopentylamine, dimethyl-(1-methylbutyl)amine, dimethyl-(2-methylbutyl)amine, dimethyl-(2,3-dimethylpropyl)amine, dimethyl-tert-pentylamine, methyldiethylamine, methylethyl-n-propylamine, methylethylisopropylamine, methylethyl-n-butylamine, methylethylisobutylamine, methylethyl-sec-butylamine, methylethyl-tert-butylamine, triethylamine, diethyl-n-propylamine, and diethylisopropylamine.

Hereinafter; the organic bas having a total of 3 to 7 carbon atoms may be simply referred to as the "organic base". Also the aliphatic or aromatic sulfonic acid may be simply referred to as the "acid".
The production method of the present invention will be described in further detail below.
An unsaturated ether compound represented by general formula (2) can be obtained by reacting an acetal represented by general formula (1) in the presence of aliphatic or aromatic sulfonic acid and an organic base having a total of 3 to 7 carbon atoms under, for example, normal pressure, reduced pressure, or increased pressure.

In the production method of the present invention, the molar ratio of the acid to the organic base is preferably 2:1 to 1:1 and more preferably 1.5:1 to 1:1. In the reaction system, the acid and the organic base may form a salt.
In the production method of the present invention, an acetal having a boiling point of 150°C or less can be used as a raw material.

The production method of the present invention can be carried out continuously or discontinuously using, for example, a stirring-type reactor, a distillation tower, and the like.
In the production method of the present invention, the amount of the organic base is not particularly limited but is preferably 0.0001 to 0.2 mol and more preferably 0.001 to 0.1 mol per mole of acetal according to the selection of apparatuses, such as the capacities of the reactor and the distillation tower.

The reaction temperature can be selected according to the pressure. In general, the reaction can be performed at the maximum temperature of 150° C or less. In some cases, the acid and the organic base remain as a residue in the reactor after the reaction when the target unsaturated ether compound is distilled from the reactor. In this case, the acid and the base remaining as the residue may be reused.
When the acetal used in the production method of the present invention contains water, the acetal may be azeotropically dehydrated with any azeotropic solvent with water, such as benzene, toluene; or ethanol, or water may be chemically consumed with an ester or an acid anhydride. Alternatively, water may be removed by physical adsorption with sodium sulfate, magnesium sulfate, or molecular sieve.

In the production method of the present invention, a reaction solvent may be used according to demand. Examples of the solvent include hydrocarbon solvents such as hexane, toluene, and xylene; ether solvents such as dioxane and tetrahydrofuran; and ketone solvents such as acetone, methyl ethyl ketone, and methyl isobutyl ketone. These solvents can be used alone or in a mixture of two or more members.

According to production method of the present invention, a simple equipment can be used, the formation of by-products and provide the target unsaturated ether compound can be produced in high yield.
The unsaturated ether compound produced by the production method of the present invention is useful for application to a chemical amplification-type resist composition, a synthetic intermediate for pharmaceuticals, a raw material for coating materials, and a hydroxyl group protecting agent which gives a hydroxyl group protector (W02003/006407, etc.).

Although the present invention is described in further detail with reference to examples, the present invention is not limited to these examples.
In the examples and comparative example, the liquid composition of the distillate and the conversion ratio were calculated by gas chromatographic analysis under the following conditions:
Apparatus used: GC-17A (manufactured by Shimadzu Corporation)
Column used: DB-1 (nonpolar column, column internal diameter 0.25 mm, column length 30 m, film thickness 0.25 µm) (manufactured by J & W Scientific Inc.)
Carrier gas: N₂, column inlet pressure: 60 kPa, gas flow rate: 40 ml/min. split ratio: 1:50

### EXAMPLE 1

### Production of 1-methoxy-2-methylpropene

In a 1 L round-bottom flask provided with a stirrer, a thermometer, and a distillation tower (corresponding to seven stages), 57.0 g of p-toluenesulfonic acid monohydrate and 27.3 g of triethylamine were mixed with 590 g of 1,1-dimethoxy-2-methylpropane. Then, the resultant mixture was heated to obtain methanol and 1-methoxy-2-methylpropene as reaction products by distillation. The distillation took place by heating to a temperature of 90°C under normal pressure. The amount of the distillate obtained was 430 g, and the distillate had a composition (measured by gas chromatographic analysis) containing 13.0% of methanol, 0.1% of isobutylaldehyde, 0.7% of 1,1-dimethoxy-2-methylpropane, and 85.8% of 1-methoxy-2-methylpropene. The conversion ratio was 98.9%. Triethylamine was not detected in the distillate. As a result of water washing as post-treatment, 98.5% of 1-methoxy-2-methylpropene was produced.

### EXAMPLE 2

### Production of 1-(1-methylethoxy)-2-methylpropene

In a 300 mL round-bottom flask provided with a stirrer, a thermometer, and a distillation tower (corresponding to seven stages), 7.53 g of p-toluenesulfonic acid monohydrate and 3.61 g of triethylamine were mixed with 216 g of 1,1-(1-methylethoxy)-2-methylpropane. Then, the resultant mixture was heated to obtain isopropyl alcohol and 1-(1-methylethoxy)-2-methylpropene as reaction products by distillation. The distillation took place by heating to a temperature of 130°C under normal pressure. The amount of the distillate obtained was 189 g, and the distillate had a composition (measured by gas chromatographic analysis) containing 14.6% of isopropyl alcohol, 2.4% of isobutylaldehyde, 2.3% of 1,1-(1-methylethoxy)-2-methylpropane, and 78.2% of 1-(1-methylethoxy)-2-methylpropene. The conversion ratio was 95.2%. Triethylamine was not detected in the distillate.

### EXAMPLE 3

### Production of 1-methoxy-2-methylpropene

In a 1 L round-bottom flask provided with a stirrer, a thermometer, and a distillation tower (corresponding to seven stages), 57.0 g of p-toluenesulfonic acid monohydrate, 27.3 g of triethylamine, and 45.1 g of trimethyl orthoformate were mixed with 590 g of 1,1-dimethoxy-2-methylpropane. Then, the resultant mixture was heated to obtain methanol and 1--methoxy-2-methylpropene as reaction products by distillation. The distillation took place by heating to a temperature of 90°C under normal pressure. The amount of the distillate obtained was 4.20 g, and the distillate had a composition (measured by gas chromatographic analysis) containing 9.3% of methanol, 2.7% of 1,1-dimethoxy-2-methylpropane, and 88.0% of 1-methoxy-2-methylpropene. The conversion ratio was 97.3%. Triethylamine was not detected in the distillate.

### Industrial Applicability

The present invention provides a method for producing an unsaturated ether compound capable of suppressing the formation of by-products and producing the unsaturated ether compound in a high yield.

## Claims

1. A method for producing an unsaturated ether compound represented by general formula (2): 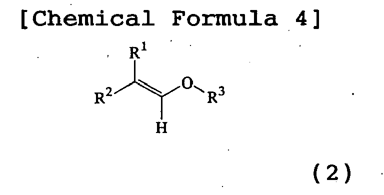 (wherein R¹, R², and R³ may be the same or different and each represent substituted or unsubstituted alkyl, substituted or unsubstituted aryl, or substituted or unsubstituted aralkyl, or R¹ and R² form cycloalkyl together with the adjacent carbon atom), which comprises reacting an acetal represented by general formula (1): 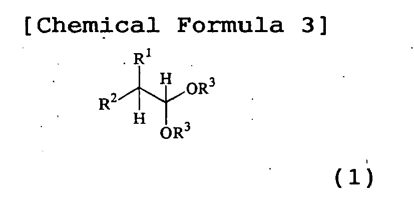 (wherein R¹, R², and R³ are each defined as the same as the above) in the presence of an aliphatic or aromatic sulfonic acid and an organic base having a total carbon number 3 to 7.

2. The method according to claim 1, wherein the organic base is a tertiary amine.
